(19) Europäisches Patentamt
European Patent Office
Office européen des brevets

(11) **EP 3 078 390 B1**

(12) **EUROPÄISCHE PATENTSCHRIFT**

(45) Veröffentlichungstag und Bekanntmachung des Hinweises auf die Patenterteilung:
**27.10.2021   Patentblatt 2021/43**

(51) Int Cl.:
***A61M 60/00*** *(2021.01)*

(21) Anmeldenummer: **16162936.5**

(22) Anmeldetag: **30.03.2016**

(54) **FLUIDFÖRDERUNGSÜBERWACHUNGSVERFAHREN IN EINER EXTRAKORPORALEN BLUTBEHANDLUNGSVORRICHTUNG**

FLUID TRANSPORT MONITORING METHOD IN AN EXTRACORPOREAL BLOOD TREATMENT DEVICE

PROCEDE DE SURVEILLANCE D'ACHEMINEMENT DE FLUIDE DANS UN DISPOSITIF DE TRAITEMENT DE SANG EXTRACORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.04.2015   DE 102015105323**

(43) Veröffentlichungstag der Anmeldung:
**12.10.2016   Patentblatt 2016/41**

(73) Patentinhaber: **B. Braun Avitum AG**
**34212 Melsungen (DE)**

(72) Erfinder:
• **Ritter, Kai-Uwe**
**91126 Rednitzhembach (DE)**

• **Schleicher, Christian**
**36160 Dipperz (DE)**

(74) Vertreter: **Winter, Brandl - Partnerschaft mbB**
**Alois-Steinecker-Straße 22**
**85354 Freising (DE)**

(56) Entgegenhaltungen:
WO-A1-2013/057109      DE-A1-102006 011 346
DE-A1-102008 039 022      DE-A1-102010 033 241
DE-A1-102011 108 778      DE-A1-102012 007 412

• **None**

## Beschreibung

Technisches Gebiet

Die Erfindung betrifft ein Verfahren zum Überwachen einer Förderung von Fluid, insbesondere Blut, in einer Vorrichtung zur extrakorporalen Blutbehandlung, insbesondere in einer Dialysemaschine, wobei Fluid mittels einer Peristaltikpumpe in einem Fluidsystem von einer Niederdruckseite zu einer Hochdruckseite gefördert wird und eine elastisch verformbare und zwischen der Niederdruckseite und der Hochdruckseite angeordnete Fluidleitung zwischen einer Stützfläche, insbesondere einer durch ein Gehäuse der Peristaltikpumpe gebildeten Stützfläche, und zumindest zwei Quetschelementen eines gegenüber der Stützfläche rotierenden Rotors derart verformt wird, dass zwischen den Quetschelementen, insbesondere zwischen unmittelbar aufeinanderfolgenden oder unmittelbar zueinander benachbarten Quetschelementen, ein Fördervolumenabschnitt ausgebildet wird, wobei für den Fördervolumenabschnitt ein fördervolumenspezifischer Fluiddruckwert im Fluidsystem erfasst wird.

Stand der Technik

Bekannte Peristaltikpumpen bei Medizingeräten zur extrakorporalen Blutbehandlung bestehen in der Regel aus einem Rotor, einem Pumpengehäuse und einer zwischen diesen angeordneten Schlauchleitung als Fluidleitung, und fördern ein definiertes Volumen eines Mediums, wie zum Beispiel Blut oder Dialysefluid, von der Niederdruckseite, in der Regel die arterielle Seite, auf die Hochdruckseite, in der Regel die venöse Seite. Die Peristaltikpumpe arbeitet durch Verformen und Abklemmen der elastisch verformbaren Fluidleitung mittels der Quetschelemente. Diese sind gegen die Fluidleitung vorgespannt und werden bei Rotation des Rotors an dieser entlang bewegt, wobei es zu elastischer Verformung der Fluidleitung in Querschnittsrichtung kommt. Infolge dessen wird Fluid in Förderrichtung aus der Fluidleitung herausgedrückt. Nachfließendes Fluid wird durch Niederdruck, insbesondere Vakuum, der aufgrund elastischer Rückformung der Fluidleitung nach der Verformung durch die Quetschelemente entsteht, in die Leitung hineingezogen.

Es ist bekannt, das Vorspannen der Quetschelemente gegen die Fluidleitung beispielsweise mittels Federn zu realisieren. Diese sind so ausgelegt, dass der Querschnitt der Fluidleitung vollständig abgeklemmt werden kann, anders ausgedrückt der Querschnitt der Fluidleitung mittels der Quetschelemente vollständig verschlossen werden kann. Druckdifferenzen werden daher bei ordnungsgemäß funktionierender Pumpe nicht von der arteriellen Seite auf die venöse Seite und umgekehrt übertragen.

Hochdruckseitig entsteht in der Regel beim Öffnen des Fördervolumens, also beim Übergang des Fluidleitungsquerschnitts von okklusiv auf nicht-okklusiv, Pulsation. Dieser Effekt kommt daher, dass niederdruckseitig ein Volumenabschnitt der elastischen Fluidleitung abgeklemmt wird, und das darin eingeschlossene Fluidvolumen durch Rotation des Rotors und dadurch bedingte Verlagerung der Quetschstelle der Fluidleitung in Richtung der Hochdruckseite gefördert wird. Bei Abklemmen des Fördervolumenabschnitts steht das darin eingeschlossene Volumen unter niederdruckseitigem Druck. Es wird unter diesem Druck auf die Hochdruckseite gefördert, wo hingegen Hochdruck herrscht. Wird im Rahmen des Fördervorgangs der Fördervolumenabschnitt zur Hochdruckseite geöffnet, kommt es aufgrund der Druckdifferenz zwischen der Hochdruckseite und dem Fördervolumenabschnitt zu einer Fluidströmung von der Hochdruckseite in den Fördervolumenabschnitt. Diese Rückströmung dauert an, bis Druckausgleich vorliegt. Folge ist ein kurzzeitiges Einbrechen des hochdruckseitigen Drucks und es kommt zu Pulsation auf der Hochdruckseite.

Im Falle einer Förderung von Blut als Fluid kann es während des vorstehend beschriebenen Druckausgleichs wie auch infolge eines nicht vollständig mittels der Quetschelemente verschlossenen Fluidleitungsquerschnitts (unzulängliche Abdichtung des Fördervolumenabschnitts) sein, dass Blut durch die dabei vorliegende oder entstehende Engstelle zwischen dem Fördervolumenabschnitt und der Hochdruckseite gequetscht wird. Dabei kommt es in der Regel zur teilweisen Zerstörung von Blutzellen, allgemein als Hämolyse bezeichnet. Zur Vermeidung von Hämolyse werden die Quetschelemente, die Fluidleitung und die Stützfläche der Peristaltikpumpe derart aufeinander abgestimmt, dass sich der Querschnitt der Fluidleitung bei Öffnen des Fördervolumenabschnitts zur Hochdruckseite möglichst schnell erweitert, also der Übergang zwischen den Zuständen okkludiert und nicht okkludiert möglichst schnell stattfindet und des Weiteren der Querschnitt während der Förderung möglichst dicht verschlossen ist.

Es ist ein Nachteil bekannter Verfahren zum Fördern von Fluid, insbesondere von Blut, dass es im Verlauf der Einsatzdauer einer derartigen Peristaltikpumpe zu Verschleiß oder Versagen kommen kann, wodurch ein vollständiges Verschließen des Querschnitts der Fluidleitung und ein rascher Übergang von okkludiert zu nicht-okkludiert nicht sichergestellt werden kann. Hämolyse kann daher nicht sicher ausgeschlossen werden. Es ist weiterhin von Nachteil, dass ein Versagen einer ein Quetschelement gegen die Fluidleitung vorspannenden Feder bei bekannten Verfahren nur erkannt werden kann, wenn es derart vollständig erfolgt, dass erforderliche Drücke nicht mehr aufgebaut werden können. Ein nicht erkanntes Versagen der vorstehend beschriebenen Art kann einen Patienten unter Umständen massiv schädigen, da erhöhte Hämolyse entstehen kann.

Gattungsgemäßer Stand der Technik ist etwa aus den Dokumenten DE 10 2008 039022 A1, DE 10 2010

# EP 3 078 390 B1

033241 A1, DE 10 2011 108778 A1, DE 10 2006 011346 A1, WO 2013/057109 A1, DE 10 2012 007412 A1 bekannt.

## Offenbarung der Erfindung

Ausgehend von dem vorstehend beschriebenen Stand der Technik liegt der vorliegenden Erfindung die Aufgabe zugrunde, die zuvor angeführten Nachteile zu beseitigen, insbesondere ein Verfahren zum Überwachen einer Förderung von Fluid sowie im weitesten Sinne ein Verfahren zum Fördern von Fluid zu schaffen, bei dem unvollständiges Verschließen des Querschnitts der Fluidleitung und ein verzögerter Übergang von okkludiert zu nicht-okkludiert, insbesondere Verschleiß oder Fehler, sicher und schnell erfasst werden können, so dass das Risiko von Hämolyse minimiert werden kann.

Nach der Erfindung wird diese Aufgabe gelöst durch ein Verfahren zum Überwachen einer Förderung von Fluid in einer Vorrichtung zur extrakorporalen Blutbehandlung oder ein Verfahren zum Fördern von Fluid in einer solchen Vorrichtung, wobei Fluid mittels einer Peristaltikpumpe in einem Fluidsystem von einer Niederdruckseite zu einer Hochdruckseite gefördert wird, indem eine elastisch verformbare und zwischen der Niederdruckseite und der Hochdruckseite angeordnete Fluidleitung zwischen einer Stützfläche und zumindest zwei Quetschelementen eines gegenüber der Stützfläche rotierenden Rotors derart verformt wird, dass zwischen den Quetschelementen ein Fördervolumenabschnitt ausgebildet wird, wobei für den Fördervolumenabschnitt ein fördervolumen(abschnitts)spezifischer Fluiddruckwert im Fluidsystem erfasst wird und wobei ein Förderzyklus jeweils eine vollständige Umdrehung des Rotors umfasst, der erfasste fördervolumen(abschnitts)spezifische Fluiddruckwert eines Förderzyklus n mit dem erfassten fördervolumen(abschnitts)spezifischen Fluiddruckwert eines vorangegangenen Förderzyklus n-x verglichen wird.

Mit dem erfindungsgemäßen Verfahren kann in besonders vorteilhafter und einfacher Weise mangelnder Verschluss des Querschnitts der Fluidleitung erfasst werden. Dieser kann zum Beispiel infolge von Verschleiß oder Versagen der Quetschelemente und/oder diese gegenüber der Fluidleitung vorspannende Elemente, wie zum Beispiel Federn, entstehen. Des Weiteren kann mit dem erfindungsgemäßen Verfahren Verschleiß oder Alterung der Fluidleitung, z.B. Erschlaffen oder Abknicken, insbesondere auf der arteriellen Seite, erfasst werden. Schließlich kann mit dem Verfahren sichergestellt werden, dass Fluid derart gefördert wird, dass ein Öffnen des Fördervolumenabschnitts bei Auslaufen eines Quetschelements aus der Förderstrecke, also der Übergang von okkludiert zu nicht-okkludiert, bestmöglich abgestimmt ist auf eine Vermeidung von Rückströmung von Fluid von der Hochdruckseite in den Fördervolumenabschnitt bei gleichzeitig nicht zu starkem Anpressen der Quetschelemente an die Fluidleitung, um übermäßigen Verschleiß zu vermeiden. Es ist nun möglich, zur Vermeidung von Hämolyse die Quetschelemente und die Stützfläche, auch als Pumpenbett bezeichnet, so aufeinander abzustimmen, dass während der Rotation der Übergang zwischen den Zuständen okkludiert und nicht-okkludiert möglichst schnell stattfindet. Wenn okkludiert ist, kann sichergestellt sein, dass möglichst dicht und fortwährend okkludiert ist. Andererseits kann sichergestellt sein, dass nicht zu fest okkludiert wird, um Verschleiß im Pumpensegment zu minimieren.

Das erfindungsgemäße Verfahren wird vorzugsweise durchgeführt mit Bezug auf eine Dialysemaschine bzw. mittels einer Dialysemaschine mit einer Peristaltikpumpe zur Förderung von Fluid. Die Peristaltikpumpe weist eine elastisch verformbare Fluidleitung, zum Beispiel einen Schlauch, eine die Fluidleitung stützende Stützfläche, insbesondere ausgebildet durch ein Pumpengehäuse, und einen Rotor auf. Die Fluidleitung ist zwischen der Niederdruckseite und der Hochdruckseite platziert und verbindet diese Seiten strömungstechnisch miteinander. In die Fluidleitung eintretendes Fluid stammt von der Niederdruckseite, aus der Fluidleitung austretendes Fluid strömt in die Hochdruckseite. In der Regel ist die Niederdruckseite die arterielle Seite und die Hochdruckseite die venöse Seite. Der Rotor weist zumindest zwei Quetschelemente auf. Jedes Quetschelement verformt bei Rotation des Rotors die Fluidleitung zwischen sich und der Stützfläche. Im Sinne der vorliegenden Beschreibung umfasst der Begriff "Förderzyklus" jeweils eine vollständige Umdrehung des Rotors, wobei eine Umdrehung als Förderzyklus n bezeichnet wird. Die dem Förderzyklus n folgende Umdrehung des Rotors wird als Förderzyklus n+1 und weitere folgende Umdrehungen als Förderzyklen n+2, n+3, etc. bezeichnet. Die Bezeichnung "Förderzyklus n+x" bezeichnet einen beliebigen und nicht näher spezifizierten Förderzyklus der dem Förderzyklus n nachfolgenden Förderzyklen. Die dem Förderzyklus n vorangegangene Umdrehung des Rotors wird als Förderzyklus n-1 und weitere vorangegangene Umdrehungen als Förderzyklen n-2, n-3, etc. bezeichnet. Die Bezeichnung "Förderzyklus n-x" bezeichnet einen beliebigen der dem Förderzyklus n vorangegangenen Förderzyklen.

Der Rotor und die die elastische Fluidleitung stützende Stützfläche sind derart ausgebildet und aufeinander abgestimmt, dass zwischen ihnen eine Förderstrecke ausgebildet ist. Im Bereich der Förderstrecke wird die Fluidleitung zwischen der Stützfläche und einem Quetschelement quer zu ihrem Querschnitt verformt und bei ordnungsgemäßer Funktion im Wesentlichen fluiddicht zusammengequetscht. Ein vorlaufendes Quetschelement läuft erst aus der Förderstrecke aus, wenn ein nachlaufendes Quetschelement in die Förderstrecke eingelaufen ist. Anders ausgedrückt ist der Winkel zwischen Förderstreckeneinlauf und Förderstreckenauslauf größer als der Winkel zwischen einem vorlaufenden Quetschelement und einem nachlaufenden Quetschelement. Es gibt daher nach der Erfindung stets einen Zeitraum bzw. eine Förderstreckenabschnitt, bei dem ein zwischen vorlaufendem Quetschelement und nachlaufendem Quetschelement gefördertes Fluidvolumen zwischen diesen eingeschlossen und bei ordnungsgemäßer Funktion abgedichtet ist. Das zwischen zwei benachbarten Quetschelementen im Bereich der Förderstrecke eingeschlossene Fluidvolumen wird bei der vorliegenden Erfindungsbeschreibung als Fördervolumenabschnitt bezeichnet.

Der Rotor weist zumindest zwei Quetschelemente auf. Dies ist die geringstmögliche Anzahl an Quetschelementen, die zur Ausbildung eines definierten, insbesondere eines abgedichteten Fördervolumenabschnitts erforderlich ist. Das Verfahren nach der Erfindung kann auch mit einem Rotor ausgeführt werden, der mehr als zwei Quetschelemente aufweist, insbesondere drei oder vier. Die Winkelpositionen der Quetschelemente zueinander, also die zwischen benachbarten Quetschelementen befindlichen Winkel, betragen vorzugsweise im Falle von zwei Quetschelementen 180°, im Falle von drei Quetschelementen 120° und Falle von vier Quetschelementen 90°. Die Länge der Förderstrecke der Fluidleitung ist in allen Fällen größer. Ein Rotor mit zwei Quetschelementen A und B bildet zwei Fördervolumenabschnitte aus, einen ersten Fördervolumenabschnitt AB zwischen den Quetschelementen A und B und einen zweiten Fördervolumenabschnitt BA zwischen den Quetschelementen B und A. Ein Rotor mit drei Quetschelementen A, B und C bildet drei Fördervolumenabschnitte aus, einen ersten Fördervolumenabschnitt AB zwischen den Quetschelementen A und B, einen zweiten Fördervolumenabschnitt BC zwischen den Quetschelementen B und C und einen dritten Fördervolumenabschnitt CA zwischen den Quetschelementen C und A. In entsprechender Weise bildet ein Rotor mit vier Quetschelementen A, B, C und D vier Fördervolumenabschnitte AB, BC, CD sowie DA aus.

Nach der Erfindung wird der Fluiddruckwert eines bestimmten Fördervolumenabschnitts mit einem diesem bestimmten Fördervolumenabschnitt zugewiesenen Referenzwert in Form des erfassten fördervolumenspezifischen Fluiddruckwerts eines vorangegangenen Förderzyklus n-x verglichen. Fördervolumenspezifisch in diesem Sinne bedeutet, dass mittelbar oder unmittelbar aufeinanderfolgende Fluiddruckwerte jeweils eines Fördervolumens miteinander verglichen werden. Mit Bezug auf den vorstehenden Absatz bedeutet dies, dass ein Fluiddruckwert des Fördervolumenabschnitts AB eines Förderzyklus n mit dem aufgenommenen Fluiddruckwert dieses Fördervolumenabschnitts AB eines vorangegangenen Förderzyklus n-x verglichen wird. Gleiches gilt für weitere vorhandene Fördervolumenabschnitte. Das Verfahren nach der Erfindung umfasst sowohl eine Überwachung jedes einzelnen Fördervolumenabschnitts der jeweils zur Anwendung kommenden Pumpe wie auch eine Überwachung nur einzelner, also nicht aller Fördervolumenabschnitte. Man kann auch sagen, dass im Rahmen der Überwachung ein zu einem frühen Zeitpunkt (beim Förderzyklus n-x) bereits aufgenommener Fluiddruckwert für jeden einzelnen Fördervolumenabschnitt einen diesem zugewiesenen Referenzwert darstellt. Der Referenzwert spiegelt in den Verfahren nach der Erfindung den Normalzustand wieder, also einen Betriebszustand, zu dem sicher davon auszugehen ist, dass Fluid in der gewünschten Weise gefördert wird und die Pumpe ordnungsgemäß funktioniert. Zu Beginn des Verfahrens kann dieser Referenzwert bei neuer, unverschlissener Pumpe aufgezeichnet werden. Auf das Vorliegen eines Fehlers kann nach der der Erfindung zugrundeliegenden Idee geschlossen werden, wenn eine bestimmte Abweichung der aufgenommenen Ist-Fluiddruckwerte von dem Referenzwert auftritt. Zum Auslösen einer Fehlermeldung kann diesbezüglich ein Schwellwert festgelegt sein oder werden. Man kann also auch sagen, dass das Verfahren nach der Erfindung derart funktioniert, dass Ist-Druckwerte mit Soll-Druckwerten verglichen werden, welche Soll-Druckwerte den ordnungsgemäßen und gewünschten Zustand des Systems und des Verfahrens wiederspiegeln.

Die Quetschelemente können unmittelbar am Rotor ausgebildet sein, insbesondere einstückig mit dem Rotor. Sie können alternativ auf Rotorarmen angeordnet sein. Die Quetschelemente können insbesondere als Quetschrollen oder Andruckrollen, die in vorteilhafter Weise materialschonend an der Fluidleitung abrollen, oder Gleitschuhe, die sich gleitend über die Fluidleitung bewegen, ausgebildet sein. Die Quetschelemente sind insbesondere in radialer Richtung positionierbar und radial nach außen, also in eine die Fluidleitung zusammenquetschende Position vorgespannt. Vorzugsweise erfolgt diese Vorspannung mittels Federelementen.

Mit der Erfindung können insbesondere die folgenden Vorteile erzielt werden:

- Reduktion bzw. Vermeidung von blutschädigender Hämolyse, da mangelhaftes Verschließen des Querschnitts der Fluidleitung sowie mangelhafter Übergang von okkludiert zu nicht-okkludiert einfach und schnell erfasst werden können,
- ein Rückströmen von Fluid vom Hochdruckbereich in die Förderstrecke aufgrund von Druckdifferenz kann so verringert oder vermieden werden, was zu einer Verringerung oder gar Vermeidung von zusätzlicher Pulsation führt;
- der Vergleich von jeweils zwei Messwerten über eine zeitliche Verzögerung ermöglicht es, dass der genaue Zeitpunkt des Federbruchs erkannt werden kann;
- beim Vergleich von zwei Messwerten zweier Zyklen kann der Federbruch auch deutlich schneller erkannt werden, als bei einem Vergleich mit beispielsweise einem Referenzwert, der durch Mittelwertbildung aus einer Vielzahl an vorangegangenen Förderzyklen ermittelt wird.

Vorteilhafte Ausführungsformen der Erfindung sind in den Unteransprüchen beansprucht und werden nachfolgend näher erläutert.

Nach einer Ausführungsform ist x ein Wert zwischen 1 und 50, vorzugsweise zwischen 5 und 40, besonders bevorzugt zwischen 5 und 30, weiter bevorzugt zwischen 5 und 20 ist. Insbesondere kann es sich bei dem vorangegangenen Förderzyklus um den Förderzyklus n-1, n-2, n-3, n-4, n-5, n-6, n-7, n-8, n-9, n-10 oder noch weiter zurückliegende Förderzyklen handeln. Die zuvor angegebenen Bereiche sind derart zu verstehen, dass x jede ganze Zahl in den angegebenen Bereichen annehmen kann.

Eine Ausführungsform des Verfahrens, bei der jeder oder nahezu jeder Förderzyklus (also n-1, n-2 oder n-3) überwacht wird, funktioniert besonders gut, um plötzlich auftretende Veränderungen zu erfassen. Die dieser Ausführungsform zugrundeliegende Idee ist, dass ausgehend von einem ordnungsgemäß funktionierenden System bestimmte Parameter überwacht werden und bei einer plötzlichen Veränderung der überwachten Parameter vom Vorliegen eines Fehlers, wie zum Beispiel eines plötzlich auftretenden Federbruchs, ausgegangen wird. Bei einem ordnungsgemäß funktionierendem System ändern sich nämlich Fluiddrücke oder Fluiddruckverläufe eines Fördervolumenabschnitts von Förderzyklus zu Förderzyklus im Wesentlichen nicht. Patientenbewegung können zwar Druckänderungen hervorrufen, diese sind aber meist nur von sehr kurzer Dauer und nicht periodisch. Eine plötzliche Veränderung der überwachten Fluiddrücke bzw. Fluiddruckverläufe ist erst bei Auftreten eines Fehlers festzustellen, zum Beispiel bei nicht vollständiger Okklusion infolge Federbruch.

Eine andere Ausführungsform des Verfahrens, bei der zeitlich weiter voneinander beabstandete Förderzyklen (beispielsweise bei n-10, n-15, n-20, etc.) miteinander verglichen werden, ist besonders gut geeignet, um schleichende Prozessänderungen beispielsweise infolge von bei längerem Einsatz nach und nach auftretendem Verschleiß zu erfassen.

Nach einer weiteren Ausführungsform der Erfindung kann die Erfassung des Fluiddruckwerts im Fluidsystem in einer Erfassung des Druckverlaufs des Fluiddruckwerts bestehen. Der Druckverlauf kann einer digitalen Signalauswertung unterzogen werden. Diese Ausführungsform des Verfahrens ist besonders geeignet, um kleinere Fehler, wie zum Beispiel eine leichte Undichtigkeit, die über den gesamten Fördervolumenabschnitt vorliegt, zu erfassen.

Insbesondere kann das Leistungsdichtespektrum des erfassten Fluiddruckwerts, insbesondere des erfassten Druckverlaufs des Fluiddruckwerts, des Förderzyklus n mit dem Leistungsdichtespektrum des Referenzwerts verglichen werden. Dabei kann das Leistungsdichtespektrum jedes Fördervolumenabschnitts oder nur bestimmter ausgewählter Fördervolumenabschnitte berechnet werden. Ausgehend von Leistungsdichtespektren bei ordnungsgemäß funktionierendem System wird bei einer bestimmten Änderung der überwachten Leistungsdichtespektren auf das Vorliegen eines Fehlers geschlossen. Durch Aufzeichnen der Druckkurven und Leistungsdichtespektren ist sogar der genaue Zeitpunkt des Auftretens des Fehlers feststellbar. Denn das Leistungsdichtespektrum des ersten Fördervolumenabschnitts nach dem Fehler weicht von den Leistungsdichtespektren vorangegangener Perioden ohne diesen Fehler ab.

In einer Form des Verfahrens können die berechneten Leistungsdichtespektren mit einem angenommenen idealen bzw. früher aufgezeichneten abgespeicherten Leistungsdichtespektrum verglichen werden. Bei zu starken Abweichungen liegt eine Unregelmäßigkeit vor. Hierfür kann bei einer Form der Erfindung ein Schwellwert festgelegt sein oder werden.

Bei einer anderen Form des Verfahrens kann das Leistungsdichtespektrum eines Fördervolumenabschnitts mit dem Leistungsdichtespektrum des jeweils vorangegangenen entsprechenden Fördervolumenabschnitts verglichen werden, also z.B. das Spektrum des Fördervolumenabschnitts n+1 mit dem Spektrum des Fördervolumenabschnitts n, dann das Spektrum des Fördervolumenabschnitts n+2 mit dem Spektrum des Fördervolumenabschnitts n+1, usw..

Des Weiteren können Spektren nur entsprechender Fördervolumenabschnitte miteinander verglichen werden. Bei einer Peristaltikpumpe mit zwei Quetschelementen A und B sowie zwischen diesen liegenden Fördervolumenabschnitten AB und BA werden dabei einerseits immer die Spektren des Fördervolumenabschnitts AB miteinander verglichen, also zum Beispiel AB(n) mit AB(n+1), AB(n+1) mit AB(n+2), usw., und andererseits die Spektren der Fördervolumenabschnitte BA miteinander verglichen, also zum Beispiel BA(n) mit BA(n+1), BA(n+1) mit BA(n+2), usw.. Alternativ ist möglich, einfach die Leistungsdichtespektren aufeinanderfolgender Fördervolumenabschnitte miteinander zu vergleichen, also AB mit BA.

Nach einer Ausführungsform der Erfindung kann für den Förderzyklus n und den Förderzyklus n-x eine Korrelationsfunktion, insbesondere die Kreuzkorrelationsfunktion des Fluiddruckwerts bestimmt werden. Eine Kreuzkorrelationsfunktion $R_{xy}(\tau)$ kann zur Beschreibung der Korrelation zweier Signale x(t) und y(t) bei unterschiedlichen Zeitverschiebungen t zwischen den beiden Signalen eingesetzt werden. Es kann zum Beispiel gelten

$$R_{xy}(\tau) = \lim_{T_F \to \infty} \frac{1}{T_F} \int_{-T_F/2}^{T_F/2} x(t)\,y(t+\tau)\,dt$$

In diesem Fall ist x(t) das Drucksignal und y(t+T) das um T/q verzögerte Drucksignal, also y(t+T) = x(t+T/q), wobei q die Anzahl der Quetschelemente des Rotors ist. Die Korrelationsfunktion R wird maximal, wenn die Signale gleich sind. Im Normalfall ist zu erwarten, dass miteinander verglichene Signale ähnlich sind. Ein Schwellwert kann festgelegt werden oder sein, insbesondere nach Start der Pumpe, wenn sicher davon ausgegangen werden kann, dass diese ordnungsgemäß funktioniert. Im laufenden Betrieb kann der Wert R hinsichtlich einer Veränderung, insbesondere auf ein plötzliches Absinken hin, überwacht werden. Es kann auch eine fest vorgegebene Schwelle als Grenzwert abgespeichert sein.

Nach einer Ausführungsform der Erfindung kann der Fluiddruckwert des venösen Drucks pv erfasst werden. Nach einer anderen Ausführungsform der Erfindung kann der Fluiddruckwert $p_{BE}$ vor einem Dialysator erfasst werden. Nach einer weiteren Ausführungsform der Erfindung kann der Fluiddruckwert des arteriellen Drucks $p_A$ erfasst werden. Jede beliebige Kombination der vorgenannten Erfassungen von Druckwerten liegt außerdem im Bereich der Erfindung.

Bei allen Ausführungsformen der Erfindung kann ein Schwellwert für den erfassten Fluiddruckwert festgelegt sein oder werden, bei dessen Überschreiten ein Fehlersignal ausgegeben wird. Es ist von Vorteil, wenn ein Alarm nicht sofort nach einmaliger Grenzwertüberschreitung erfolgt, sondern mehrere Fördervolumenabschnitte beobachtet werden, um eventuell aufgenommene Störungen, z.B. durch Patientenbewegungen oder durch elektromagnetische Einflüsse auf die Sensoren, auszuschließen.

Schließlich kann eine Korrelation eines erfassten Fluiddruckwerts oder Fluiddruckwertverlaufs mit einer vordefinierten Erwartungsfunktion bestimmt werden.

Man kann auch sagen, dass die Erfindung ein Verfahren zum Betrieb einer Blutreinigungsmaschine betrifft, mit der mittels einer Blutpumpe das Blut zur Blutreinigung aus dem Körper gepumpt und nach der Blutreinigung wieder zurückgegeben wird. Die Blutpumpe kann ein Pumpentyp sein, welcher mit Hilfe von Rollen arbeitet. Diese Rollen klemmen sich mittels einer Federkraft gegen einen Schlauch als Fluidleitung in der Pumpe und bewegen sich an ihm entlang, wodurch die Flüssigkeit herausgedrückt wird. Die nachfließende Flüssigkeit wird durch den Ansaugeffekt des Vakuums, das durch die Elastizität des Schlauches entsteht, in den Schlauch hineingezogen. Das Anpressen des Schlauches durch die Rollen kann mit Hilfe von Federn realisiert werden. Die Federn können so ausgelegt sein, dass der Schlauch komplett abgeklemmt ist. Dieser abgeklemmte Schlauch dämpft alle Drucksignale und somit werden keine Druckschwankungen, welche von der arteriellen Seite hervorgerufen werden, auf die venöse Seite übertragen. Genauso können Druckschwankungen nicht von der arteriellen Seite auf die venöse Seite übertragen werden, da der geschlossene Rollenläufer diese Druckschwankungen dämpft. Mit Hilfe der Erfindung kann insbesondere eine gebrochene Feder im Rollenläufer einer Blutpumpe erkannt werden. Dieser Fehlerfall bewirkt, dass der Schlauch nicht mehr komplett abgeklemmt werden kann und verstärkt Hämolyse stattfindet. Der nicht mehr komplett abgeklemmte Schlauch bewirkt zum einen, dass Druckschwankungen, welche auf der arteriellen Seite vorliegen, nicht mehr komplett gedämpft werden und auf die venöse Seite übertragen werden. Zum anderen ruft ein nicht mehr komplett abgeklemmter Schlauch eine andere Druckschwankung bei dem Rolleneingriff bzw. Rollenausgriff hervor. Zudem würde sich das Leistungsdichtespektrum ändern (andere Frequenz würde sich einstellen). Diese beschriebenen Veränderungen können verwendet werden, um einen Federbruch zu erkennen.

Kurzbeschreibung der Zeichnungen

Die Erfindung wird im Folgenden anhand beispielhafter, nicht einschränkender und in den angehängten Figuren gezeigter Ausführungsformen näher erläutert. Dabei zeigt:

Figur 1 eine schematische Darstellung eines Ausschnitts einer Vorrichtung zur extrakorporalen Blutbehandlung,

Figur 2 eine schematische Darstellung von Fluiddruckwerten unter idealen Bedingungen,

Figur 3 eine schematische Darstellung einer Auswertung der Fluiddruckwerte der Figur 2,

Figur 4 eine schematische Darstellung einer Auswertung der Fluiddruckwerte mittels Korrelation aufeinanderfolgender Pulse, und

Figur 5 eine schematische Darstellung einer Auswertung der Fluiddruckwerte mittels Vergleich aufeinanderfolgender Pulse.

Detaillierte Beschreibung bevorzugter Ausführungsformen

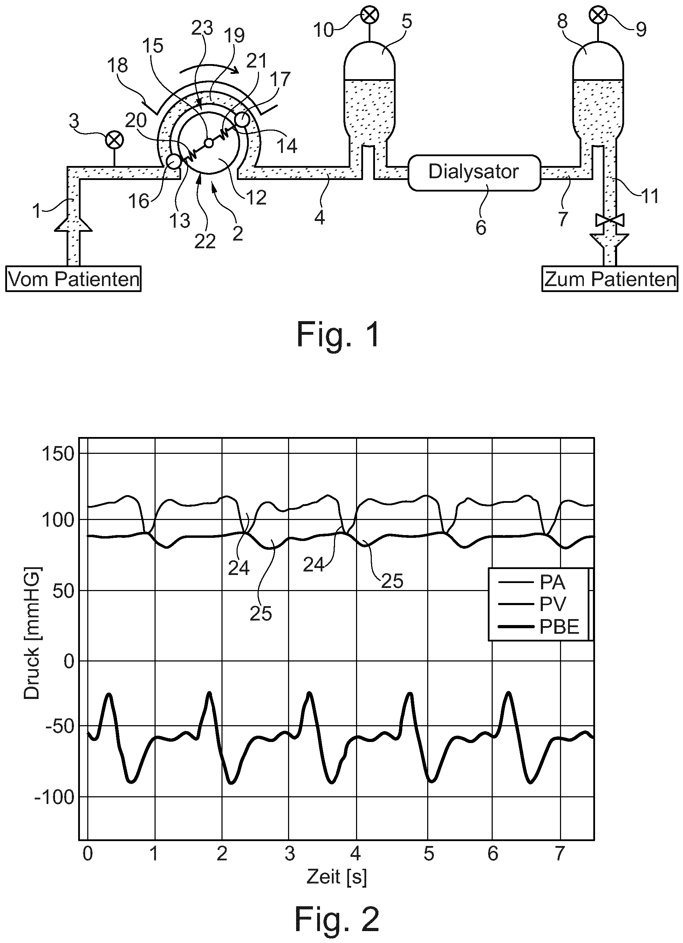

Figur 1 zeigt beispielhaft einen Ausschnitt einer Vorrichtung zur extrakorporalen Blutbehandlung, die mit dem Verfahren nach der Erfindung betrieben wird. Gezeigt ist im Wesentlichen der gesamte extrakorporale Blutkreislauf der Vorrichtung.

Niederdruckseitig weist dieser eine arterielle Blutleitung 1 auf, mittels der Blut von einem nicht gezeigten Patienten zu einer Peristaltikpumpe 2 der Behandlungsvorrichtung geführt wird. Vor der Peristaltikpumpe 2 ist ein arterieller Druckabnehmer 3 vorgesehen, mit dem der Druck vor der Peristaltikpumpe 2, also der niederdruckseitige oder arterielle Druck, gemessen wird.

Hochdruckseitig führt von der Peristaltikpumpe 2 eine Leitung 4 unter Hochdruck stehendes aber noch unbehandeltes, mit Schlackenstoffen belastetes Blut zu einer Tropfkammer 5 und von dort zu einem Dialysator 6. Diesem wird eingangsseitig Dialysierflüssigkeit zugeführt (nicht gezeigt). Im Dialysator 6 wird Blut in bekannter Weise mittels der Dialysierflüssigkeit behandelt, z.B. gereinigt. Verbrauchte Dialysierflüssigkeit wird über eine nicht gezeigte Dialysierflüssigkeitsableitung vom Dialysator 6 entfernt und einer nicht dargestellten Entsorgung oder Aufbereitung zugeführt. Behandeltes Blut wird mittels einer Blutableitung 7 vom Dialysator 6 zu einer venösen Tropfkammer 8 geleitet. An dieser ist ein venöser Druckabnehmer 9 vorgesehen, mit dem der venöse Druck, also der hochdruckseitige Druck, erfasst wird. In entsprechender Weise ist an der Tropfkammer 5 ein Druckabnehmer 10 vorgesehen, mit dem der Fluiddruck vor dem Dialysator 6 erfasst werden kann. Von der venösen Tropfkammer 8 wird behandeltes Blut über eine venöse Blutleitung 11 zurück zum Patienten geleitet.

Die Peristaltikpumpe 2 weist einen Rotor 12 mit einem ersten Rotorarm 13 und einem zweiten Rotorarm 14 auf. Die Rotorarme 13, 14 rotieren um eine gemeinsame Rotorachse 15. Der erste Rotorarm 13 trägt auf seiner der Rotorachse 15 abgewandten Seite ein erstes Quetschelement 16 in Form einer ersten Quetschrolle 16. Der zweite Rotorarm 14 trägt auf seiner der Rotorachse 15 abgewandten Seite ein zweites Quetschelement 17 in Form einer zweiten Quetschrolle 17. Die Peristaltikpumpe 2 weist des Weiteren ein in der Figur 1 nur schematisch angedeutetes Blutpumpengehäuse 18 auf, das in bekannter Weise eine Stützfläche für eine Fluidleitung 19 ausbildet. Im Blutpumpengehäuse 18 ist die elastische Fluidleitung 19 angeordnet, derart, dass sie zwischen der Stützfläche des Blutpumpengehäuses 18 und den Quetschelementen 16, 17 deformiert wird. Die elastische Fluidleitung 19 ist eingangsseitig, also niederdruckseitig, mit der arteriellen Blutleitung 1 und ausgangsseitig, also hochdruckseitig, mit der Blutleitung 4 verbunden. Sie wird mittels der Quetschelemente 16, 17 derart deformiert, dass ihr Querschnitt im fehlerfreien Normalbetrieb der Pumpe 2, wo noch kein Verschleiß vorliegt, vollständig zusammengequetscht, also im Wesentlichen fluiddicht geschlossen wird. Die Quetschrolle 16 ist mittels einer Feder 20 in Richtung der Fluidleitung 19 vorgespannt. Die Quetschrolle 17 ist mittels einer Feder 21 in Richtung der Fluidleitung 19 vorgespannt. Die Vorspannung der Federn 20, 21 ist derart ausgewählt und eingestellt, dass der Querschnitt der Fluidleitung 19 im gewünschten Maß verschlossen werden kann.

In der elastischen Fluidleitung 19 wird beim Betrieb der Pumpe 2 durch Eingreifen der Quetschelemente 16, 17 und das dadurch bedingte Zusammenquetschen des Leitungsquerschnitts zwischen dem Quetschelement 16 und dem Quetschelement 17 ein erster Fördervolumenabschnitt 22 ausgebildet. In dieser Periode läuft das vorlaufende Quetschelement 16 vor dem nachlaufenden Quetschelement 17. Ein zweiter Fördervolumenabschnitt 23 wird zwischen dem Quetschelement 17 und dem Quetschelement 16 ausgebildet. In dieser Periode läuft das vorlaufende Quetschelement 17 vordem nachlaufenden Quetschelement 16.

Figur 2 zeigt bei einem Normalbetrieb, also ohne Fehler und Verschleiß, der Peristaltikpumpe 2 der beschriebenen Vorrichtung aufgenommene Fluiddruckwerte. Aufgenommen und dargestellt sind der arterielle Fluiddruck bzw. der arterielle Fluiddruckverlauf $p_A$ (erfasst mittels des arteriellen Druckabnehmers 3), der Fluiddruck bzw. Fluiddruckverlauf vor dem Dialysator 6 $p_{BE}$ (erfasst mittels des Druckabnehmers 10) und der venöse Fluiddruck bzw. der venöse Fluiddruckverlauf pv (erfasst mittels des venösen Druckabnehmers 9). Im dargestellten Normalzustand sind beide Federn 20, 21 in Ordnung und der Querschnitt der Fluidleitung 19 wird durch Eingriff der Quetschrollen 16, 17 in der gewünschten Weise verschlossen. Der Übergang von okkludierter Fluidleitung 19 zu nicht-okkludierter Fluidleitung 19 verläuft optimal. Bei der Druckkurve des venösen Fluiddrucks pv wie auch bei der Druckkurve des Fluiddrucks $p_{BE}$ vor dem Dialysator 6 lassen sich die Quetschrollenausgriffe der Blutpumpe 2 in Form der periodisch wiederkehrenden Druckminima 24 bzw. 25 erkennen.

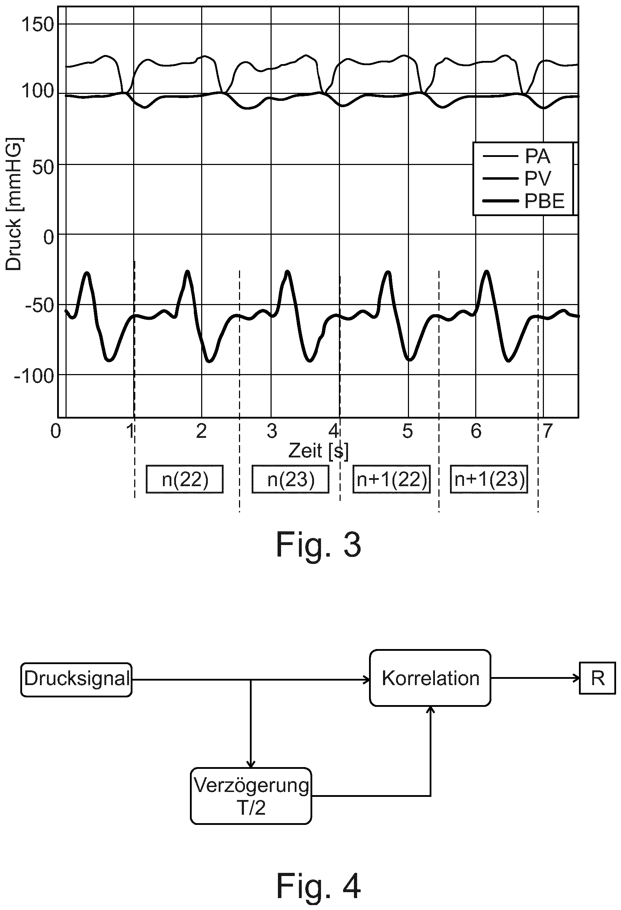

Nach der Erfindung wird genutzt, dass sich mit Hilfe einer geeigneten digitalen Signalauswertung diese Rollenausgriffe erkennen lassen. Hierzu gibt es unterschiedliche Auswerteverfahren, welche nach der Erfindung eingesetzt werden können. Figur 3 zeigt die Druckverläufe aus der Figur 2 mit periodischen Eingriffen bzw. periodischen Wertemessungen. Bei einer Form der Erfindung wird zur Auswertung der gemessenen Werte das Leistungsdichtespektrum jedes Pulses oder Fördervolumenabschnitts berechnet, z.B. mittels Fast Fourier Transformation der abgetasteten Druckverläufe. In Figur 3 ist der erste Fördervolumenabschnitt 22 eines Förderzyklus n mit n(22) bezeichnet. Der zweite Fördervolumenabschnitt 23 des Förderzyklus n mit n(23) bezeichnet. Der erste Fördervolumenabschnitt 22 eines auf den Förderzyklus n nachfolgenden Förderzyklus n+1 ist mit n+1(22) bezeichnet. Der zweite Fördervolumenabschnitt 23 des Förderzyklus n+1 mit n+1(23) bezeichnet. Figur 3 zeigt ebenfalls Druckkurven des Normalbetriebs. Es ist zu erkennen, dass für jeden Fördervolumenabschnitt ein spezifischer Signalverlauf vorliegt, zu dem ein ebenso spezifisches Leistungsdichtespektrum berechnet wird. Liegen kein Verschleiß und kein Fehler vor, wird der auf den jeweiligen Fördervolumenabschnitt 22 bzw. 23 bezogene Signalverlauf im Wesentlichen unverändert bleiben.

Bei einem Rollenausgriff mit gebrochener Feder 20 und/oder 21 wird eine andere Druckschwankung als bei einer einwandfreien Feder 20 und/oder 21 hervorgerufen und es kommt zu einer Veränderung des aufgenommenen Drucksignals, insbesondere pv und $p_{BE}$. Infolge dessen kommt es auch zu einer Veränderung des entsprechenden Leistungsdichtespektrums des betroffenen Fördervolumenabschnitts 22 bzw. 23. Durch Aufzeichnen der Druckkurven und Leistungsdichtespektren ist sogar der genaue Zeitpunkt eines Federbruchs feststellbar. Denn das Leistungsdichtespektrum des ersten Fördervolumenabschnitts nach dem Federbruch weicht von den Leistungsdichtespektren vorgegangener Perioden mit fehlerfreier Feder ab.

In einer Form des Verfahrens können die berechneten Leistungsdichtespektren mit einem angenommenen idealen bzw. früher aufgezeichneten abgespeicherten Leistungsdichtespektrum verglichen werden. Bei zu starken Abweichungen liegt eine Unregelmäßigkeit vor. Hierfür wird in der Regel ein Schwellwert festgelegt. Bei einer anderen Form des Verfahrens kann das Leistungsdichtespektrum eines Fördervolumenabschnitts mit dem Leistungsdichtespektrum des jeweils vorangegangenen Fördervolumenabschnitts verglichen, also z.B. das Spektrum des Fördervolumenabschnitts n+1(22) mit dem Spektrum des Fördervolumenabschnitts n(22), dann das Spektrum des Fördervolumenabschnitts n+2(22) mit dem Spektrum des Fördervolumenabschnitts n+1(22), usw.. Daneben wird das Spektrum des Fördervolumenabschnitts n+1(23) mit dem Spektrum des Fördervolumenabschnitts n(23), dann das Spektrum des Fördervolumenabschnitts n+2(23) mit dem Spektrum des Fördervolumenabschnitts n+1(23), usw. verglichen. Damit werden jeweils die Spektren der Fördervolumenabschnitte, die von den beiden Rollenläufern erzeugt wurden, gegeneinander verglichen. Bei zu starken Abweichungen liegt eine Unregelmäßigkeit vor. Hierfür wird in der Regel ein Schwellenwert festgelegt. Bei mehr als zwei Quetschelementen 16, 17 ist das Verfahren in der Form zu erweitern, dass die Leistungsdichtespektren der jeweiligen Fördervolumenabschnitte mit den anderen Spektren des Fördervolumenabschnitts verglichen werden. Alternativ ist es auch möglich, einfach die Leistungsdichtespektren aufeinanderfolgender Fördervolumenabschnitte miteinander zu vergleichen.

Ein anderes Auswerteverfahren, das nach der Erfindung eingesetzt werden kann, ist die in Figur 4 gezeigte Auswertung der Fluiddruckwerte mittels Korrelation aufeinanderfolgender Fördervolumenabschnitte, z.B. mittels Kreuzkorrelation. In der Signalanalyse wird die Kreuzkorrelationsfunktion Rxy($\tau$) zur Beschreibung der Korrelation zweier Signale x(t) und y(t) bei unterschiedlichen Zeitverschiebungen t zwischen den beiden Signalen eingesetzt. Es gilt

$$R_{xy}(\tau) = \lim_{T_F \to \infty} \frac{1}{T_F} \int_{-\frac{T_F}{2}}^{\frac{T_F}{2}} x(t)y(t+\tau)dt$$

In diesem Fall ist x(t) das Drucksignal und y(t+T) das um T/2 (halbe Zeit für eine Rotorumdrehung) verzögerte Drucksignal, also y(t+T) = x(t+T/2). Die Korrelationsfunktion R wird maximal, wenn die Signale gleich sind. Im Normalfall ist zu erwarten, dass die Signale sehr ähnlich sind. Ein Schwellwert kann kurz nach dem Test im Vorbereiten der Maschine festgelegt werden, wenn sicher davon ausgegangen werden kann, dass beide Federn 20 und 21 sowie die Fluidleitung 19 in Ordnung sind, also in der gewünschten Weise funktionieren. Im laufenden Betrieb wird dann kontinuierlich der Wert R hinsichtlich einer Veränderung, insbesondere auf ein plötzliches Absinken hin überwacht. Es kann auch eine fest vorgegebene Schwelle als Grenzwert abgespeichert sein.

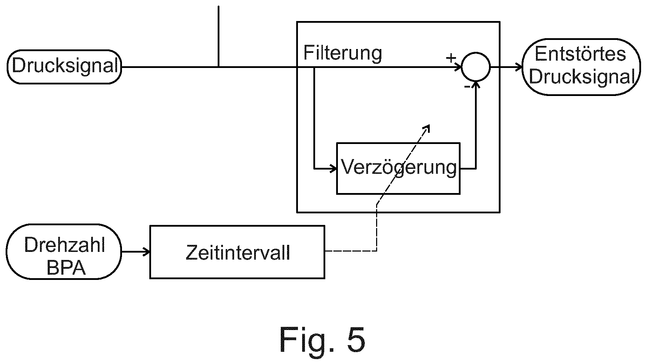

Ein drittes Verfahren zur digitalen Signalauswertung des Druckverlaufs kann im Rahmen des Verfahrens nach der Erfindung mittels Vergleich der Druckverläufe von aufeinanderfolgenden Fördervolumenabschnitten erfolgen. Figur 5 zeigt schematisch eine Verzögerung des Druckverlaufs um eine Periode (Umdrehungsgeschwindigkeit der Blutpumpe 2). Die optimale Verzögerung liegt bei einer Pumpe 2 mit zwei Quetschelementen 16, 17 bei der halben Zeit (T/2), die die Blutpumpe 2 für eine Umdrehung benötigt, also bei einer Verzögerung um einen halben Förderzyklus.

Bei einer derartigen Signalauswertung ist im Normalfall zu erwarten, dass die Signale sehr ähnlich sind, das Ergebnis am Ausgang damit nahe Null. Ein Schwellwert kann kurz nach dem Test im Vorbereiten der Maschine festgelegt werden, wenn sicher davon ausgegangen werden kann, dass beide Federn 20 und 21, sowie das Schlauchsegment 19 noch in Ordnung sind. Im laufenden Betrieb wird dann kontinuierlich der Wert auf ein plötzliches Absinken hin überwacht. Es kann auch eine fest vorgegebene Schwelle als Grenzwert abgespeichert sein.

**Patentansprüche**

1. Verfahren zum Überwachen einer Förderung von Fluid in einer Vorrichtung zur extrakorporalen Blutbehandlung,

   wobei Fluid mittels einer Peristaltikpumpe (2) in einem Fluidsystem von einer Niederdruckseite zu einer Hochdruckseite gefördert wird, indem eine elastisch verformbare und zwischen der Niederdruckseite und der Hochdruckseite angeordnete Fluidleitung (19) zwischen einer Stützfläche und zumindest zwei Quetschelementen (16, 17) eines gegenüber der Stützfläche rotierenden Rotors (12) derart verformt wird, dass zwischen den Quetschelementen (16, 17) ein Fördervolumenabschnitt (22, 23) ausgebildet wird,
   wobei für den Fördervolumenabschnitt (22, 23) ein fördervolumenspezifischer Fluiddruckwert im Fluidsystem erfasst wird, und

wobei ein Förderzyklus jeweils eine vollständige Umdrehung des Rotors (12) umfasst,

**dadurch gekennzeichnet, dass**

der erfasste fördervolumenspezifische Fluiddruckwert eines Förderzyklus n mit dem erfassten fördervolumenspezifischen Fluiddruckwert eines vorangegangenen Förderzyklus n-x verglichen wird.

2. Verfahren nach Anspruch 1, wobei x ein Wert zwischen 1 und 50 ist.

3. Verfahren nach Anspruch 1, wobei x ein Wert zwischen 5 und 40 ist.

4. Verfahren nach Anspruch 1, wobei x ein Wert zwischen 5 und 30 ist.

5. Verfahren nach Anspruch 1, wobei x ein Wert zwischen 5 und 20 ist.

6. Verfahren nach einem der vorstehenden Ansprüche, wobei der Druckverlauf des erfassten Fluiddruckwerts einer digitalen Signalauswertung unterzogen wird.

7. Verfahren nach einem der vorstehenden Ansprüche, wobei für den Förderzyklus n und den Förderzyklus n-x die Kreuzkorrelationsfunktion des Fluiddruckwerts bestimmt wird.

8. Verfahren nach einem der vorstehenden Ansprüche, wobei der Fluiddruckwert des venösen Drucks $p_V$ erfasst wird.

9. Verfahren nach einem der vorstehenden Ansprüche, wobei der Fluiddruckwert $p_{BE}$ vor einem Dialysator (6) erfasst wird.

10. Verfahren nach einem der vorstehenden Ansprüche, wobei der Fluiddruckwert des arteriellen Drucks $p_A$ erfasst wird.

11. Verfahren nach einem der vorstehenden Ansprüche, wobei ein Schwellwert für den erfassten Fluiddruckwert festgelegt ist oder wird, bei dessen Überschreiten ein Fehlersignal ausgegeben wird.

12. Verfahren nach einem der vorstehenden Ansprüche, wobei eine Korrelation mit einer vordefinierten Erwartungsfunktion bestimmt wird.

**Claims**

1. Method for monitoring a conveyance of fluid in a device for extracorporeal blood treatment,

   whereby fluid is conveyed by means of a peristaltic pump (2) in a fluid system from a low-pressure side to a high-pressure side in that an elastically deformable fluid line (19) positioned between the low-pressure side and the high-pressure side is deformed between a support surface and at least two squeeze elements (16, 17) of a rotor (12) rotating opposite the support surface in such a way that a conveyance volume section (22, 23) is formed between the squeeze elements (16, 17),

   whereby a fluid pressure value in the fluid system is detected for the conveyance volume section (22, 23) which is specific to the conveyance volume section, and

   whereby a conveyance cycle comprises a respective full rotation of the rotor (12),

   **characterized in that**

   the detected conveyance-volume-specific fluid pressure value of a conveyance cycle n is compared with the detected conveyance-volume-specific fluid pressure value of a preceding conveyance cycle n-x.

2. Method according to Claim 1, whereby x is a value between 1 and 50.

3. Method according to Claim 1, whereby x is a value between 5 and 40.

4. Method according to Claim 1, whereby x is a value between 5 and 30.

5. Method according to Claim 1, whereby x is a value between 5 and 20.

**6.** Method according to one of the preceding claims, whereby the pressure progression of the detected fluid pressure value is subjected to a digital signal analysis.

**7.** Method according to one of the preceding claims, whereby the cross-correlation function of the fluid pressure value is determined for the conveyance cycle n and the conveyance cycle n-x.

**8.** Method according to one of the preceding claims, whereby the fluid pressure value of the venous pressure $p_V$ is detected.

**9.** Method according to one of the preceding claims, whereby the fluid pressure value $p_{BE}$ is detected before a dialyzer (6).

**10.** Method according to one of the preceding claims, whereby the fluid pressure value of the arterial pressure $p_A$ is detected.

**11.** Method according to one of the preceding claims, whereby a threshold value is or has been defined for the detected fluid pressure value at the exceedance of which an error signal is emitted.

**12.** Method according to one of the preceding claims, whereby a correlation is determined with a predefined expectation function.


**Revendications**

**1.** Procédé de surveillance d'un refoulement de fluide dans un dispositif de traitement extracorporel du sang,

dans lequel le fluide est refoulé au sein d'un système de fluide au moyen d'une pompe péristaltique (2) depuis un côté basse pression vers un côté haute pression du fait qu'une conduite de fluide (19) élastiquement déformable agencée entre le côté basse pression et le côté haute pression est déformée entre une surface d'appui et au moins deux éléments d'écrasement (16, 17) d'un rotor (12) tournant par rapport à la surface d'appui de telle manière qu'une section de volume de refoulement (22, 23) est formée entre les éléments d'écrasement (16, 17),
dans lequel une valeur de pression de fluide spécifique au volume de refoulement est détectée au sein du système de fluide pour la section de volume de refoulement (22, 23), et
dans lequel un cycle de refoulement comprend respectivement un tour complet du rotor (12),
**caractérisé en ce que,**
la valeur détectée de pression de fluide spécifique au volume de refoulement d'un cycle de refoulement n est comparée à la valeur détectée de pression de fluide spécifique au volume de refoulement d'un cycle de refoulement n-x précédent.

**2.** Procédé selon la revendication 1, dans lequel x est une valeur comprise entre 1 et 50.

**3.** Procédé selon la revendication 1, dans lequel x est une valeur comprise entre 5 et 40.

**4.** Procédé selon la revendication 1, dans lequel x est une valeur comprise entre 5 et 30.

**5.** Procédé selon la revendication 1, dans lequel x est une valeur comprise entre 5 et 20.

**6.** Procédé selon l'une quelconque des revendications précédentes, dans lequel le profil de pression de la valeur détectée de pression de fluide est soumis à une évaluation numérique de signal.

**7.** Procédé selon l'une quelconque des revendications précédentes, dans lequel la fonction de corrélation croisée de la valeur de pression de fluide est déterminée pour le cycle de refoulement n et le cycle de refoulement n-x.

**8.** Procédé selon l'une quelconque des revendications précédentes, dans lequel la valeur de pression de fluide de la pression veineuse $p_V$ est détectée.

**9.** Procédé selon l'une quelconque des revendications précédentes, dans lequel la valeur de pression de fluide $p_{BE}$

est enregistrée en amont d'un dialyseur (6).

10. Procédé selon l'une quelconque des revendications précédentes, dans lequel la valeur de pression de fluide de la pression artérielle $p_A$ est détectée.

11. Procédé selon l'une quelconque des revendications précédentes, dans lequel une valeur de seuil, dont le dépassement provoque l'émission d'un signal d'erreur, est spécifiée ou doit être spécifiée pour la valeur de pression de fluide détectée.

12. Procédé selon l'une quelconque des revendications précédentes, dans lequel une corrélation avec une fonction d'anticipation prédéfinie est déterminée.

Fig. 1

Fig. 2

Fig. 3

Fig. 4

Fig. 5

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008039022 A1
- DE 102010033241 A1
- DE 102011108778 A1
- DE 102006011346 A1
- WO 2013057109 A1
- DE 102012007412 A1